# EUROPEAN PATENT APPLICATION

(11) **EP 3 806 100 A1**
(43) Date of publication of application: **14.04.2021**
(21) Application number: 19863884.3
(22) Date of filing: 09.05.2019
(51) Int. Cl.: G16H 30/20

(54) **METHOD AND DEVICE FOR FOUR-DIMENSIONAL VISUALIZATION OF MEDICAL IMAGE**

(30) Priority: 21.09.2018 CN 201811104506
(71) Applicant: Raycan Technology Co., Ltd. (Suzhou), Suzhou New District Suzhou, Jiangsu 215163 (CN)
(72) Inventor: LI, Jing, Suzhou, Jiangsu 215163 (CN); LI, Zhen, suzhou, Jiangsu 215163 (CN); DENG, Huchuan, suzhou, Jiangsu 215163 (CN); ZHU, Qian, suzhou, Jiangsu 215163 (CN); XIE, Qingguo, suzhou, Jiangsu 215163 (CN); XIAO, Peng, suzhou, Jiangsu 215163 (CN); ZHANG, Zhi, suzhou, Jiangsu 215163 (CN); GAO, Jielin, suzhou, Jiangsu 215163 (CN); YANG, Yi, suzhou, Jiangsu 215163 (CN)
(74) Representative: Grauel, Andreas
(86) International application number: PCT/CN2019/086231
(87) International publication number: WO 2020/057141

(57) **Abstract**

The present application provides a method and device for four-dimensional visualization of medical images, the medical image data is uploaded to the control module by the user via the client, the medical image data is analyzed and the analyzing result is fed back to the client by the control module, the medical image data is preprocessed by the storage server to form the cross-sectional tomographic images, dynamic three-dimensional image display is provided on the client by the processing module according to the cross-sectional tomographic images. The device comprises perception module, control module, communication module and processing module, the control module communicates with the client through the interconnection unit, the storage server communicates with the control module and the cache server respectively, the cache server communicates with the control module, the processing module communicates with the storage server, the cache server and the control module respectively. The modules of the present application can be synchronized quickly, the data access performance is improved, the operation is convenient, the expansibility is strong, the consumptions of computing resources of the network transmission data and the client are reduced, and the cost is lower.

## Description

### FIELD OF THE INVENTION

The present application relates to an image processing method and device in the field of medical devices, in particular to a method and device for four-dimensional visualization of medical images.

### BACKGROUNDOF THE INVENTION

With the rapid development of the computer technology, the communication technology and the medical imaging technology, the medical image visualization technology has gradually been transformed from standalone mode to network mode and digital mode, and the way that merely relies on traditional two-dimensional planar medical images for clinic diagnosis cannot meet the needs of doctors. On this basis, the research of three-dimensional medical image visualization technology has been developed rapidly. The three-dimensional medical image visualization technology can provide doctors with more objective relevant information required for diagnosis, such as information showing the organ morphology, the blood vessels distribution and trend, the size and spatial location of the focus of diseases and the relationships with other tissues or organs around and so on. This information can make up the limitation and subjectivity of artificial diagnosis by doctors, which can enable doctors to observe and analyze medical images from more angles and aspects and obtain the information contained in medical images more intuitively, which plays a very important role in diagnosis and treatment of diseases.

The method of three-dimensional visualization of medical images mainly comprises: (1) the way of remote modeling and rendering and local interacting and display, in which the corresponding interactive events and required rendering and modeling parameters are mainly obtained through the page in the local browser, and then this data is sent to the remote modeling and rendering side for rendering; (2) the way of remote modeling and local rendering and interacting, in which a remote modeling side and a local rendering side, the difference between which and the remote modeling and rendering is that it does not need data rendering and only needs to build a data model generated by medical image data preprocessing, the local rendering side can render and interact after obtaining the data model from the remote modeling side; (3) the way of local browser medical image visualization, which can visualize medical images on the local browser directly.

At present, there are not many methods for three-dimensional visualization of medical images based on Browser/Server (i.e. B/S) mode framework. For example, Mahmoudi proposes a Web-Based medical image visualization framework, and realizes an extensible two-dimensional and three-dimensional medical image visualization platform, wherein the internal components of the platform are independent of each other and realize image preprocessing, registration, segmentation and visualization. However, it takes up a lot of internet bandwidth when running high-frequency interaction and high computing load visualization algorithm. Hachaj uses a method of greatly reducing the resolution in the B/S mode framework to realize direct volume rendering. Although the network overhead of the method is relatively low, the overall frame rate is also relatively low and the clarity of the image frame in the interaction process is relatively poor. Wangkaoom et al use the technologies of HTML5, JavaScript, WebGL (Web Graphics Library, a 3D drawing protocol) and so on to realize high-quality real-time volume rendering framework. However, when the amount of data is relatively large, the time for data transmission and initialization is relatively long, and the interaction fluency is relatively low. In a word, the methods for medical image visualization in clinic mainly have the following disadvantages:
First, because of the large amount and high computing demand of medical image data and the fact that the image processing device in the prior art needs to use high-performance graphic workstation, the processing cost is high and the efficiency is low and the processing often needs to depend on large-scale imaging equipment, and thus the processing process is limited by time and place.
Second, the medical image processing system in the prior art, which is not easy to operate and learn, often needs to be operated by a specially assigned person.
Third, the methods used by different image processing systems are quite different, so that the equipment supplier needs to appoint professional engineers to complete the maintenance and update of the system, which is very inconvenient.
Fourth, the medical image processing system in the prior art has single function and has the limitation of medical image format, which is difficult to meet the needs of modern medicine in precision and individuation.

### SUMMARY OF THE INVENTION

The purpose of the present application is to provide a method and device for four-dimensional visualization of medical images, so as to overcome the problem that the medical image processing system in the prior art cannot meet the needs of four-dimensional imaging in precision and individuation in modern medicine.

Thus, a method for four-dimensional visualization of medical images according to the present application is provided, which comprises:
Step S1: uploading medical image data to a control module by at least one client;
Step S2: analyzing the medical image data by the control module and feeding back an analyzing result to the client, and transferring the medical image data and the analyzing result to a storage server simultaneously;
Step S3: preprocessing the medical image data with the storage server and sending formed preprocessed images to a processing module;
Step S4: conducting dynamic three-dimensional image processing according to the preprocessed images by the processing module and providing three-dimensional dynamic display of medical images on the client.

According to an embodiment of the present application, in the step S1, the medical image data is transmitted to the control module in forms of compressed data and messages by the client.

According to an embodiment of the present application, in the step S1, user requests are sent to the control module by the client via an interconnection unit, the user requests are received and the analyzing result is fed back to the client by the control module, and different user requests are distributed to the storage server and the processing module by the control module simultaneously.

According to an embodiment of the present application, in the step S1, types of the medical image data comprise: file formats of Digital Imaging and Communications in Medicine, RawData, Statistical Parametric Mapping, Neuroimaging Informatics Technology Initiative, Interfile, Analyze, Portable network graphics, MINC, BMP, and JPEG.

According to an embodiment of the present application, in the step S2, the medical image data is analyzed by the control module via a central server, data interaction between the control module and the client is accomplished via the central server, and the medical image data and the analyzing result are transferred to the storage server by the central server via a communication module.

According to an embodiment of the present application, the communication module comprises a first communication sub module, a second communication sub module and a third communication sub module which communicate with each other, the first communication sub module communicates with the interconnection unit by the central server, the storage server communicates with the first communication sub module by the third communication sub module, and the processing module communicates with the central server and the storage server by the second communication sub module.

According to an embodiment of the present application, in the Step S3, the preprocessing comprises: cross-sectional tomographic images is generated in three orthogonal directions of X, Y and Z respectively by the processing module according to three-dimensional data of the medical image data, and the cross-sectional tomographic images are saved to the storage server.

According to an embodiment of the present application, in the step S4, reconstruction result of each frame is calculated by the processing module via a rendering core sub module, communication and message analyzing between the processing module and the client and database operation between the processing module and a cache server is responsible by the processing module via a network communication sub module, and interaction events from the client are responded, interactive request messages from the network communication sub module are classified and sorted, and incorrect or repeated interactive request messages are discarded by the processing module via a logical management sub module.

According to an embodiment of the present application, in the step S4, processing result is transmitted to the client in forms of compressed data and messages by the processing module after dynamic three-dimensional image processing is conducted for dynamic display of medical images.

A device for four-dimensional visualization of medical images using above method for four-dimensional visualization of medical images is also provided by the present application, the device comprising:
a perception module, comprising an interconnection unit and at least one client that communicates with the interconnection unit;
a control module, which communicates with the client via the interconnection unit to process various user requests from the client;
a communication module, comprising a first communication sub module, a second communication sub module and a third communication sub module which communicate with each other, wherein the first communication sub module communicates with the interconnection unit via the control module; and
a processing module, comprising a storage sub module and a logical sub module, the storage sub module comprising a storage server and a cache server, wherein the storage server communicates with the cache server via the third communication sub module, the cache server communicates with the first communication sub module, the second communication sub module and the third communication sub module respectively, and the logical sub module communicates with the storage server and the control module via the second communication sub module.

According to an embodiment of the present application, the control module comprises several control units which communicate with each other and process different user requests respectively.

According to an embodiment of the present application, one of the control units is a central server, the central server communicates with the client via the interconnection unit.

According to an embodiment of the present application, the first communication sub module, the second communication sub module and the third communication sub module are respectively arranged in the central server, the logical sub module and the storage server.

According to an embodiment of the present application, a medical image file uploaded by the client is received and analyzed by the central server of the control module, and the medical image file and an analyzing result are saved to the storage server.

According to an embodiment of the present application, types of the medical image file comprises: file formats of Digital Imaging and Communications in Medicine, RawData, Statistical Parametric Mapping, Neuroimaging Informatics Technology Initiative, Interfile, Analyze, Portable Network Graphics, MINC, BMP, and JPEG.

According to an embodiment of the present application, segmentation, registration, modeling and reconstruction processing are performed on the medical image file according to the user requests by the logical sub module.

According to an embodiment of the present application, cross-sectional tomographic images are extracted according to the medical image file by the storage sub module and the cross-sectional tomographic images are stored to the storage server.

According to an embodiment of the present application, the logical sub module comprises rendering core sub module, network communication sub module and logical management sub module which communicate with each other, wherein reconstruction calculation is performed on each frame of medical image file by the rendering core sub module, information is fed back to the client and interactive request messages are sent to the logical management sub module by the network communication sub module, and the interactive request messages are classified and sorted, and useless or repeated interactive request messages are eliminated by the logical management sub module.

According to an embodiment of the present application, the control module, the communication module and the processing module are all constructed on the same server side, the client and the server side are independent of each other, and the client communicates with the server side via the control module and the communication module.

According to an embodiment of the present application, medical image data is transmitted between the client and the server side in the forms of compressed data and messages.

According to an embodiment of the present application, user requests are sent to the control module by the client via the interconnection unit, the user requests are received and data is fed back to the client by the control module, and different user requests are distributed to the storage server, the cache server and the logical sub module by the control module simultaneously.

According to an embodiment of the present application, the client comprises a mobile phone, a tablet and a desktop computer.

According to an embodiment of the present application, the storage sub module and logical sub module in the processing module are formed by using a number of servers communicatively connected with each other, and the storage sub module and the logical sub module are constructed on different servers and operate independently of each other.

According to an embodiment of the present application, the processing module is provided with a resource monitoring sub module which communicates with the interconnection unit in the perception module.

According to an embodiment of the present application, the cache server is a key-value storage database.

The method and device for four-dimensional visualization of medical images provided by the present application have the following advantages:
First, the cache server is used as the data exchange center to extend system architecture and the master-slave synchronization is supported, which enables the data to be synchronized quickly among the perception module, communication module, processing module and control module and thus greatly improves the performance of data access.
Second, the user can perform each processing operation by common computer(s) or even mobile device(s) directly without installing the system locally.
Third, by designing the system architecture as different modules, the system is divided into four modules, wherein the perception module feeds back the interactive behavior of the user, the communication module is responsible for the communication between modules, the processing module is responsible for the storage, distribution and processing of medical image data, such as segmentation, registration, modeling and four-dimensional visualization, and the control module processes all kinds of requests from the perception module, manage user rights, distribute server side resources, and display the processing results of medical images. This device has great strong expansion ability, and different medical image visualization methods can be quickly deployed to it.
Fourth, with the least possible network transmission data and client computing resource consumption, the four-dimensional visualization of multiplanar reconstruction, volume reconstruction, surface reconstruction and so on of medical images is realized, the intuitive and accurate four-dimensional images are reconstructed, the display speed of the image is accelerated as much as possible, and the medical image files are transformed into pre-processed images of specific formats so that the web page display of the medical image files is realized, wherein the preprocessed images are lossless compressed without loss of data.
Fifth, the cost is lower and the device can be accessed at any time, any place and any platform, which is more flexible and can bring greater convenience to doctors and researchers.

### BRIEF DESCRIPTION OF THE DRAWINGS

These and other features and advantages of this application will become more apparent to those skilled in the prior art from the detailed description of preferred embodiment. The drawings that accompany the description are described below.
Fig. 1 is a schematic diagram showing an arrangement of a device for four-dimensional visualization of medical images in accordance with a preferred embodiment of the present application.
Fig. 2 is a schematic diagram showing the medical image file hierarchy of the device for four-dimensional visualization of medical images in accordance with Fig. 1.
Fig. 3 is a flow diagram of the processing manage database of the device for four-dimensional visualization of medical images in accordance with Fig. 1.
Fig. 4 is a flow diagram of the Redis communication mode of the device for four-dimensional visualization of medical images in accordance with Fig. 1.
Fig. 5 is a flow diagram of the medical image file analyzing of the method for four-dimensional visualization of medical images in accordance with Fig. 1.
Fig. 6 is a schematic diagram showing processing results of the logical sub model of the device for four-dimensional visualization of medical images in accordance with Fig. 1.

### DESCRIPTION OF THE PREFERRED EMBODIMENTS

The followings are used to further illustrate the present application with specific embodiments. It should be understood that the following embodiments is only used to explain the present application but not to limit the scope of the present application.

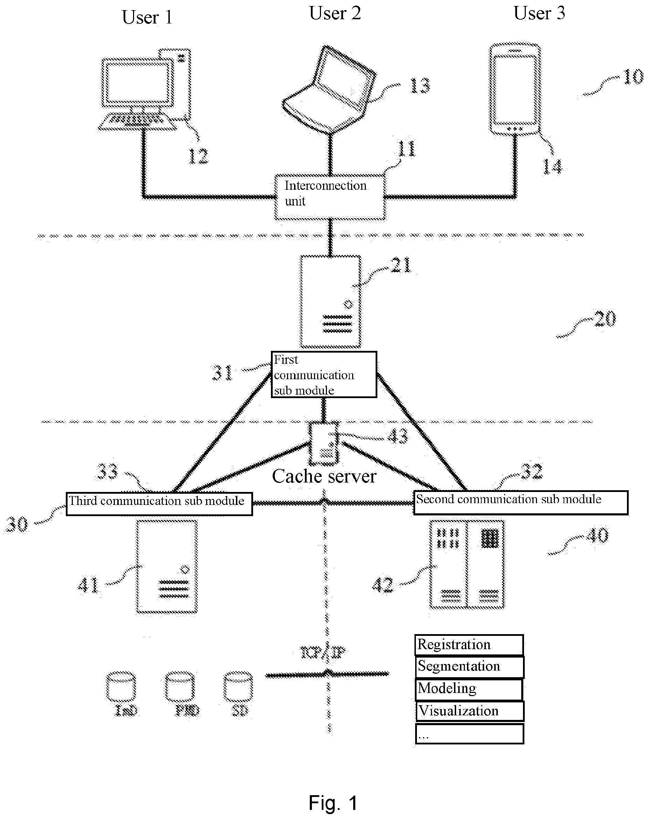

Fig. 1 is a schematic view showing an arrangement of a device for four-dimensional visualization of medical images provided by the present application. It can be seen form Fig. 1 that the device for four-dimensional visualization of medical images provided by the present application comprises a perception module 10, a control module 20, a communication module 30 and a processing module 40, wherein the perception module 10 comprises an interconnection unit 11 and several clients 12, 13, 14, the control module 20 comprises a central server 21 which communicates with clients 12, 13, 14 separately through the interconnection unit 11,the communication module 30 includes a first communication sub module 31, a second communication sub module 32 and a third communication sub module 33 which communicate with each other, the processing module 40 includes a storage sub module and a logical sub module 32, the storage sub module includes a storage server 41 and a cache server 43, and the first communication sub module 31, the second communication sub module 32 and the third communication sub module 33 are respectively arranged in the central server 21, the logical sub module 42 and the storage server 41, so as to provide communication between various modules of the entire device. More specifically, the first communication sub module 31 communicates with the interconnection unit 11 through the central server 21, the third communication sub module 33 in the storage server 41 communicates with the second communication sub module 32, the first communication sub module 31 and the cache server 43 separately, the second communication sub module 32 communicates with the first communication sub module 31, and the cache server 43 communicates with the first communication sub module 31, the second communication sub module 32 and the third communication sub module 33 separately. The perception module 10 feeds back the interactive behavior that is conducted by the user through the clients 12, 13, 14. The control module 20 is the core of the entire device, which connects with the storage server 41, the cache server 43, the logical sub module 42 and the clients through the first communication sub module 31 in the central server 21 and controls the operations of all servers. The control module 20 processes various kinds of requests from the perception module 10, manages user rights, distribute server side resources, and display processing results of medical images. The communication module 30 is responsible for the communication between various modules. The storage server 41 is responsible for the storage and distribution of the data, which provides an important database guarantee for the normal operation of the device. The cache server 43 provides aids for the rapid data exchange and synchronization of the device. The processing module 40 is responsible for the processing of medical images, such as segmentation, registration, modeling and reconstruction, and it provides a carrier for the applications of complex algorithms and visualization.

More specifically, in the embodiment of Fig. 1, the control module 20 is responsible for receiving the user request sent from the perception module 10, analyzing the user request, judging the validity and type of the user request, obtaining data from the storage sub module as needed and distributing it to different clients 12, 13, 14 or forwarding the user request to the processing module 40. After the user uploads original medical image data to the control module 20 through the perception module 10, the control module 20 first stores the uploaded medical image data in the database of the storage server 41 and parses the medical image data at the same time. After the analyzing of the medical image data is completed, the control module 20 returns the analyzing result to the perception module 10 to inform the user of the uploading state of medical image data. If the uploading state is normal, an image data processing is further carried out. After the image data processing is completed, the analyzing result is returned to the perception module 10 to mark that the uploaded file is available. After the uploading and analyzing is completed, the uploaded medical images will be displayed on web pages in one dimension or multi-dimensions through the perception module 10. Since medical image data usually has tens of megabytes or even thousands of megabytes, when transmitting medical image data, in order to realize the rapid transmission of a large number of medical data, the control module 20 exchanges data with the cache server 43 through the first communication sub module 31. In addition, in order to realize multi-user simultaneous operation, the sever needs to be equipped with a processor with relatively many cores. Therefore, in terms of hardware, the control module 20 generally adopts a physical machine equipped with a processor with at least 4 cores and a 10-gigabitdual optical interface network card to support the rapid transmission of medical data and the multi-user simultaneous operation above.

The storage sub module mainly realizes the following functions: the storage server 41 in the storage sub module is responsible for the pre-processing of medical image data to form the pre-processed images, as well as the storage of the medical image files and database, and the cache server 43 in the storage sub module provides aids for the rapid data exchange and synchronization of the device. The specific types of the medical image files include: file formats of DICOM (Digital Imaging and Communications in Medicine, which is the international standard ISO 12052 for medical image and related information), RawData, SPM (Statistical Parametric Mapping), NIFTI (Neuroimaging Informatics Technology Initiative), Interfile, Analyze, Portable network graphics, MINC, BMP, and JPEG and so on. In terms of the hardware environment, the storage server 41 uses the array card to store the disk array and regularly back up the data, and uses the database management tool to build a database which needs to store the following information: website dynamic information, file storage path, and system usage record and provide the corresponding service.

The processing module 40 is the main processing platform of image visualization, which is responsible for the processing of medical images, such as segmentation, registration, modeling and reconstruction and so on. The processing module 40 obtains data from the cache server 43 under the management of the control module 20, and in addition, it receives the user request from the perception module 10, parses the request and judges the validity of the request, and processes it accordingly, and sends the processing result to the control module 20. In addition, the processing module 40 is equipped with a resource monitoring program, which is used to monitor the usage of the resources such as CPU, GPU, memory, video memory and so on and send the corresponding data to the control module 20 so that the control module 20 can schedule tasks according to the usage of resources. In terms of hardware, the processing module 40 can use multiple high-performance physical machines according to the demand, wherein the storage sub module can be composed of one or more storage servers each of which is equipped with at least 16T storage space so as to realize storage needs of a large number of medical image data. The logical sub module is equipped with a processor with at least 12 cores, a 64G memory, a 6G dual graphics card, a 10G dual optical interface network card, which is used to meet a large number of requirements for computing and data interaction, and all the physical machine configurations can be expanded according to the actual needs.

The cache server 43 is the data exchange center of the high-speed network channel, which can be used as the high-speed exchange channel and buffer among the central server 21, the storage server 41 and the logical sub module 42. The cache server 43 is a key-value storage database (also known as key-value memory database) which has very high speed of reading and writing data and remains the easy-using of the database at the same time compared with other databases based on disk, so it can be used as a buffer. In terms of hardware, the cache server 43 can be integrated with the storage server 41 and the logical sub module 42 on the same physical machine, or use another physical machine alone. In the embodiment of Fig. 1, the cache server 43 and the storage server 41 are integrated on the same physical machine.

The device for four-dimensional visualization of medical images provided by the present application is based on B/S architecture, takes the cache server 43 as the exchange center of data, and is set up by three servers to separate algorithm, data and interface, which has the advantages of low coupling, fast deployment and high maintenance, etc. and can be used on browsers (PC and mobile device) without any extension plug-in. Since the data storage and the execution and processing are completed on the storage module 41 and the logical sub module 42 separately, the data need to communicate between different servers, and the data communication between servers has extremely high requirements on transmission efficiency. In the case of massive concurrency and large load flow, besides the data transmission in the physical media, both the reading and writing and the encapsulation of files will affect the communication efficiency. The device uses the master-slave connection mode of the cache server 43 to realize the "synchronization" of data, instead of the traditional "send/receive" mode of files, so as to ensure the efficiency of data transmission and processing.

According to the above-mentioned system layout, the corresponding database and communication method are needed to be designed to support the operation of the separated architecture and to process and visualize medical images based on B/S architecture. The complete separation of data and processing can make the hardware of the server used to the full.

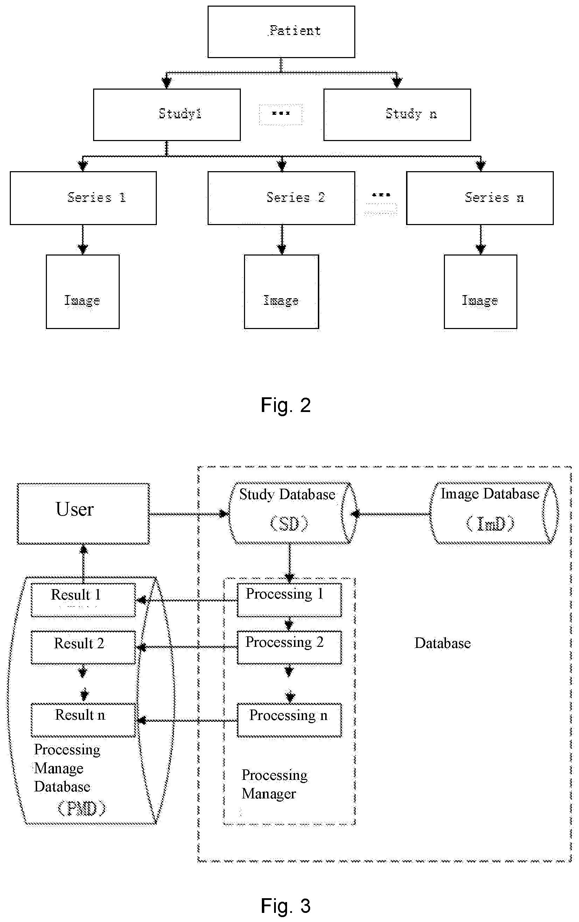

Fig. 2 is a schematic diagram showing the medical image file hierarchy of the device for four-dimensional visualization of medical images in accordance with Fig. 1.lt can be seen from Fig.1 and Fig. 2 that the database in the present application mainly comprises: (1) Study Database (SD for short), for storing the information of patients; (2) Image Database (ImD for short), for storing the image information of patients; (3) Processing Manage Database (PMD for short), for storing the management information of the processing process. These three kinds of databases are set in the storage server 41, which make the entire device have the capability of data mining and retrospective analysis. In the present application, the objects that the perception module 10 sends to the processing module 40 through the control module 20 and the communication module 30 are medical image files, which include four levels of medical image information: patient, study, series and image, and each level has a key value that can uniquely identify the hierarchical attribute. The central server 21 of the control module 20 parses the medical image files, decomposes each medical image file, and takes the extensible markup language file as the study information of the medical image files. The image pixel information, the original medical image data and the preprocessed image constitute the image information of the medical image files. The study information and the image information are both stored in the storage server 41 through the first communication sub module 31 and the third communication sub module 33 and form the Study Database (SD) and the Image Database (ImD).

Fig. 3 is a flow diagram of the processing manage database of the device for four-dimensional visualization of medical images in accordance with Fig. 1.lt can be seen from Fig. 2 and Fig. 3 that the Processing Manage Database (PMD) is also formed in the storage server 41, and the Processing Manage Database communicate with the perception module 10 and the Study Database (SD) separately. The user sends requests to the control module 20 through the perception module 10, and the processing module 40 will record every processing process of each study processed in the Study Database and send it to the Processing Manage Database to store when the control module 20 sends different user requests to the processing module 40 through the communication module 30.The user can also obtain different records of processing results through sending user requests. The Processing Manage Database makes the system have the capability of data mining and retrospective analysis and enables the system to be no longer limited to some simple operations such as window transformation and enhancement, but combined with a variety of image processing methods for more complex image processing operations. The processing process information recorded in the Processing Manage Database includes: the operator of the processing, the sequence/image processed, the processing time, the processing method, the processing parameter and the processing result, wherein each kind of processing process information has a unique "type" value and corresponding parameter string in the device. The Processing Manage Database stores the result file achieved by processing and records the address of the result file.

When the user uses the system, the data needs to be transmitted not only from the processing module 40 to the perception module 10, but also within different modules. Since the control module 20 and the storage sub module and the logical sub module 42 of the processing module 40 are physically separated, the device uses three communication modes of AJAX (i.e. Asynchronous Javascript and XML), WebSocket (a network protocol, which was defined by IETF as standard RCF6455 in 2011) and Redis (a key-value storage system) to complete data exchange according to the different communication contents and communication environments between different modules and the perception module 10, wherein the cache server 43 is used for rapid data synchronization to realize the transmission of data in high efficiency, so as to quickly respond to the processing requirements of the perception module 10.

As for a AJAX communication mode, it can realize the polling communication between the processing module 40 and the perception module 10, that is to say, the perception module 10 sends the AJAX requests to the processing module 40at regular intervals, the processing module 40 returns the response data to the perception module 10 after receiving the AJAX request, and then closes the connection. After the perception module 10 receives the response data, the Javascript in the clients 12, 13 and 14 will process the response data and update it in the page files of clients. The present application uses this communication mode to transmit two-dimensional medical image between the perception module 10 and the processing module 40, which has a relatively high stability and compatibility, and reduces the consumption of the bandwidth of the processing module and time during image transmission as much as possible. Although AJAX communication mode can realize the data transmission between the perception module 10 and the processing module 40, when the data exchange is very frequent, such as when the three-dimensional data is reconstructed in real time in the medical image processing and the processing module 40 needs to send the processing result to the perception module 10 in real time and actively, AJAX communication mode cannot meet the requirement. Therefore, further, the present application uses a WebSocket communication mode. WebSocket is a new network protocol based on TCP, which can realize the full-duplex communication between the browser and the server, i.e., it allows the processing module 40 to send information to the perception module 10 actively. In addition, WebSocket defines a series of interfaces to realize the push function between modules, which can greatly reduce the consumption of network bandwidth and so on in real time data transmission and improve the performance of the system.

During connection process of WebSocket communication mode, a WebSocket connection request is sent by the perception module 10, and the processing module 40 sends response after receiving the request message. This process completes a handshaking between the perception module 10 and the processing module 40 and builds an information channel between them so that the data information can be transmitted at any time without connecting multiple times and waiting for response. Compared with AJAX communication mode, in terms of message push, the processing module 40 no longer returns the data after receiving the request message from the perception module 10passively.Instead, when there is new request message data, the processing module 40 will actively pushes it to the processing module 40.In some real time processing processes of medical images in the present application, the processing module 40 needs to continuously render the processing results at a relatively high frame rate, this data will be continuously sent to the perception module 10 by WebSocket communication mode instead of by continuously sending requests by the perception module 10. In terms of real-time massive data transmission, WebSocket communication mode can greatly reduce the consumption of network bandwidth and has performance advantages.

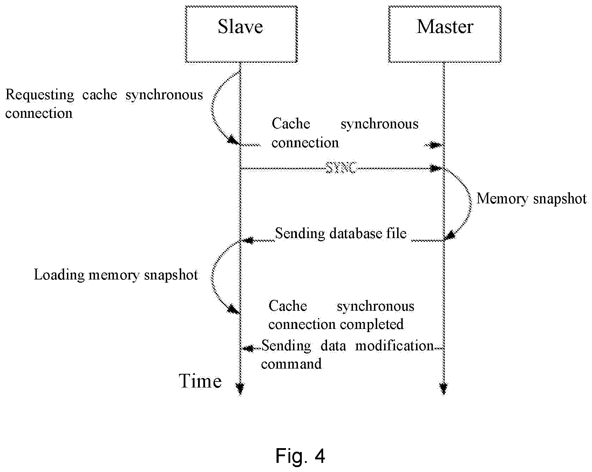

Fig. 4 is a flow diagram of the Redis communication mode of the device for four-dimensional visualization of medical images in accordance with Fig. 1.lt can be seen from Fig. 4 that the data synchronization between the processing module 40 and the storage server 41 is conducted through the master-slave connection, wherein the processing module 40 is acts as the Master, and the storage server 41 acts as the Slave, and a segment of memory of the Master is directly copied to the memory of the Slave through optical fiber without the intermediate processes of file analyzing, hardware reading and writing and so on. (1) After building the connection between the Master and Slave through "request cache synchronous connection" state, the Slave will sends a SYNC (i.e. cache synchronous connection) command to the Master actively; (2) after receiving the SYNC command, the processing module 40 of the Master starts to call the BGSAVE instruction (which is used to save the data of current database to disk asynchronously in the background) to write the data of the Master to a database file (rdb file), and uses the cache server 43 to cache all subsequent modification instructions for modifying the dataset; (3) after executing the BGSAVE command, the processing module 40 of the Master sends the snapshot file to the storage server 41 of the Slave, and keeps executing dataset modification instructions during sending; (4) after receiving the snapshot file, the storage server 41 of the Slave discards all previous old data and loads the snapshot file received; (5) after sending the snapshot file, the processing module 40 of the Master starts to send the dataset modification instructions of the buffer to the storage server 41 of the Slave in the format of Redis protocol; (6) after completing the loading of the snapshot, the storage server 41 of the Slave starts to receive the command requests and execute the dataset modification instructions from the buffer of the Master. After that, the Master continues to transmit all the collected data modification instructions and new data modification instructions to the Slave in turn, and the Slave will execute these data modification instructions again to achieve the final data synchronization. Redis communication mode has a very high data operation speed, wherein it can read about 110000 pieces of data per second, write about 80000 pieces of data per second and support rich data type operations such as String, Lists, Hashes and so on. The results of the processing process are all stored in the memory of the processing module 40 and can be quickly saved in the storage server 41 through the data synchronization of the cache server 43, and can be further sent to the user through the control module 20 as needed. This communication mode can avoid the frequent reading/writing of image processing results of the server side on the hard disk and realize the rapid data exchange and synchronization between the memories.

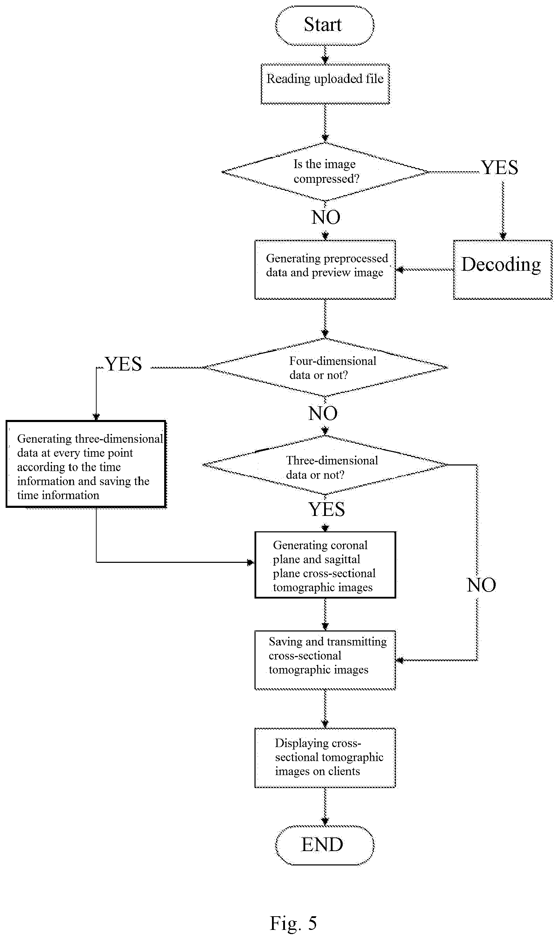

Fig. 5 is a flow diagram of the medical image file analyzing of the method for four-dimensional visualization of medical images in accordance with Fig. 1.lt can be seen from Fig. 5 that when the present application is used, the user can select different clients to upload the medical image file to the central server 21 as needed, and then the central sever 21 will parse the medical image file and transfer the medical image file and the analyzing result to the storage server 41. After processing, the user can view the relevant medical image file information and preview image in the data list page provided by the clients. As for four-dimensional medical images, it is necessary to parse the time information of the medical images first and save it to the Image Database (ImD) of the storage server 41, and then let the storage server 41 preprocess the images at different time points and provide cross-sectional tomographic images with time information in three directions of transverse section, coronal plane and sagittal plane. Then the processing module provides the clients with dynamic three-dimensional medical image visualization function through the control module 20 according to the time information and cross-sectional tomographic image information. As for three-dimensional medical images, after the uploading and analyzing of the medical image file are completed, the storage server 41 will preprocess the medical image file and provide cross-sectional tomographic images in three directions of transverse section, coronal plane and sagittal plane, and the processing module will provide the clients with the display function of the medical images through the control module 20 according to the cross-sectional tomographic images. Specifically, as shown in Fig. 5, after the medical image file is uploaded, the storage server will make the cross-sectional tomographic images in three orthogonal directions of X, Y and Z respectively by using the three-dimensional data of the medical image file, and save them that are the preprocessed images to the hard disk of the storage server 41.The preprocessed images are in three formats: (1) if medical image file images are two-dimensional data in X and Y directions, they will only be saved as a group of lossless preprocessed images in the order of z-axis; if they are three-dimensional data, they will be saved as three groups of lossless preprocessed images in three different directions of X, Y and Z; if they are four-dimensional data, the information of time points will be recorded according to the recorded time value of T, and then for each time point T, they will be saved as "T*3" groups of lossless preprocessed images in three different directions of X, Y and Z. (2) If image data of the medical image file is in the image format of 8-bit depth, it will be directly saved as 8-bit lossless preprocessed image, which will support 256 index color channels at most. (3) If image data of the medical image file is in the image format of16-bit, 24-bit or 32-bit depth, the medical image data of medical images will be put into the RGBA (red, green, blue, alpha) channels of the preprocessed images from high to low according to the order of the occupation size, which will support about 16 million color channels. After the processing, the user can view images of the medical image file through the clients. The using of the preprocessed images in the present application has advantages of not occupying the CPU performance of the server, low delay time of image transmission, strong adaptability, and a good supporting condition for browser. The preprocessed images of the present application are in one of the commonly used image formats and are supported by all common browsers.

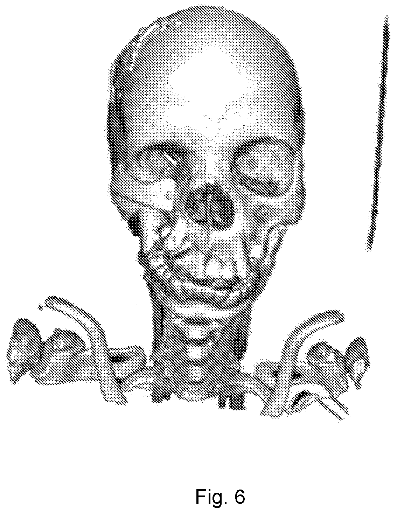

When the present application conducts the four-dimensional visualization of medical images, the user sends the four-dimensional visualization request to the processing module 40 through the WebServers of the clients 12, 13 and 14, wherein the logical sub module 42 of the processing module 40 comprises three sub modules: (1) the rendering core sub module, which is a core processing module and responsible for calculating the reconstruction result of each frame; (2) the network communication sub module, which is responsible for the communication and message analyzing between it and the clients and the database operation between it and the cache server 43, and sending the interactive request messages to the logical management sub module 42; (3) the logical management sub module, which is responsible for responding to interaction events from the clients, classifying and sorting the interactive request messages from the network communication sub module and discarding the incorrect or repeated interactive request messages to reduce the computing burden of the rendering core sub module. The logical sub module 42 of the present application can realize the operations such as Volume Rendering (VR), Surface Reconstruction (SR), Maximal Intensity Projection (MIP) and Multi-Planner Reformation (MPR) and so on, so as to further dynamically generate the three-dimensional image group with a time element, i.e., to realize the four-dimensional visualization of medical images. Fig. 6 is the three-dimensional medical image processed by the logical sub module 42 of the device for four-dimensional visualization of medical images provided by the present application. It can be seen from Fig. 6 that the three-dimensional medical image obtained by present application is processed quickly and displayed clearly.

The method and device for four-dimensional visualization of medical images provided by the present application have the following advantages:
First, the cache server is used as the data exchange center to extend system architecture and the master-slave synchronization is supported, which enables the data to be synchronized quickly among the perception module, communication module, processing module and control module and thus greatly improves the performance of data access.
Second, the user can perform each processing operation by common computer(s) or even mobile device(s) directly without installing the system locally.
Third, through the modular system architecture design, the system is divided into four modules, wherein the perception module feeds back the interactive behavior of the user, the communication module is responsible for the communication between modules, the processing module is responsible for the storage, distribution and processing of medical image data, such as segmentation, registration, modeling and four-dimensional visualization, and the control module processes all kinds of requests from the perception module, manage user rights, distribute server side resources, and display the processing results of medical images. This device has great strong expansion ability, and different medical image visualization methods can be quickly deployed to it.
Fourth, with the least possible network transmission data and client computing resource consumption, the four-dimensional visualization of multiplanar reconstruction, volume reconstruction, surface reconstruction and so on of medical images is realized, the intuitive and accurate four-dimensional images are reconstructed, the display speed of the image is accelerated as much as possible, and the medical image files are transformed into pre-processed images of specific formats so that the web page display of the medical image files is realized, wherein the preprocessed images are lossless compressed without loss of data.
Fifth, the cost is lower and the device can be accessed at any time, any place and any platform, which is more flexible and can bring greater convenience to doctors and researchers.

The foregoing application has been described in accordance with the relevant legal standard, thus the description is exemplary rather than limiting in nature. Variations and modifications to the disclosed embodiment may become apparent to those skilled in the prior art and do come within the scope of the application. Accordingly, the scope of legal protection afforded this application can only be determined by studying the following claims.

## Claims

1. A method for four-dimensional visualization of medical images, comprising:
Step S1: uploading medical image data to a control module by at least one client;
Step S2: analyzing said medical image data by said control module and feeding back an analyzing result to said client, and transferring said medical image data and said analyzing result to a storage server simultaneously;
Step S3: preprocessing said medical image data with said storage server and sending formed preprocessed images to a processing module;
Step S4: conducting dynamic three-dimensional image processing according to said preprocessed images by said processing module and providing three-dimensional dynamic display of medical images on said client.

2. The method for four-dimensional visualization of medical images according to claim 1, wherein in said step S1, said medical image data is transmitted to said control module in forms of compressed data and messages by said client.

3. The method for four-dimensional visualization of medical images according to claim 1, wherein in said step S1, user requests are sent to said control module by said client via an interconnection unit, said user requests are received and said analyzing result is fed back to said client by said control module, and different user requests are distributed to said storage server and said processing module by said control module simultaneously.

4. The method for four-dimensional visualization of medical images according to claim 1, wherein in said step S1, types of said medical image data comprise: file formats of Digital Imaging and Communications in Medicine, RawData, Statistical Parametric Mapping, Neuroimaging Informatics Technology Initiative, Interfile, Analyze, Portable network graphics, MINC, BMP, and JPEG.

5. The method for four-dimensional visualization of medical images according to claim 1, wherein in said step S2, said medical image data is analyzed by said control module via a central server, data interaction between said control module and said client is accomplished via said central server, and said medical image data and said analyzing result are transferred to said storage server by said central server via a communication module.

6. The method for four-dimensional visualization of medical images according to claim 5, wherein said communication module comprises a first communication sub module, a second communication sub module and a third communication sub module which communicate with each other, said first communication sub module communicates with said interconnection unit by said central server, said storage server communicates with said first communication sub module by said third communication sub module, and said processing module communicates with said central server and said storage server by said second communication sub module.

7. The method for four-dimensional visualization of medical images according to claim 1, wherein in said Step S3, said preprocessing comprises: cross-sectional tomographic images is generated in three orthogonal directions of X, Y and Z respectively by said processing module according to three-dimensional data of said medical image data, and said cross-sectional tomographic images are saved to said storage server.

8. The method for four-dimensional visualization of medical images according to claim 1, wherein in said step S4, reconstruction result of each frame is calculated by said processing module via a rendering core sub module, communication and message analyzing between said processing module and said client and database operation between said processing module and a cache server are accomplished by said processing module via a network communication sub module, and interaction events from said client are responded, interactive request messages from said network communication sub module are classified and sorted, and incorrect or repeated interactive request messages are discarded by said processing module via a logical management sub module.

9. The method for four-dimensional visualization of medical images according to claim 1, wherein in said step S4, processing result is transmitted to said client in forms of compressed data and messages by said processing module after dynamic three-dimensional image processing is conducted for dynamic display of medical images.

10. A device for four-dimensional visualization of medical images, comprising:
a perception module, comprising an interconnection unit and at least one client that communicates with said interconnection unit;
a control module, which communicates with said client via said interconnection unit to process various user requests from said client;
a communication module, comprising a first communication sub module, a second communication sub module and a third communication sub module which communicate with each other, wherein said first communication sub module communicates with said interconnection unit via said control module; and
a processing module, comprising a storage sub module and a logical sub module, said storage sub module comprising a storage server and a cache server, wherein said storage server communicates with said cache server via said third communication sub module, said cache server communicates with said first communication sub module, said second communication sub module and said third communication sub module respectively, and said logical sub module communicates with said storage server and said control module via said second communication sub module.

11. The device for four-dimensional visualization of medical images according to claim 10, wherein said control module comprises several control units which communicate with each other and process different user requests respectively.

12. The device for four-dimensional visualization of medical images according to claim 11, wherein one of said control units is a central server, said central server communicates with said client via said interconnection unit.

13. The device for four-dimensional visualization of medical images according to claim 12, wherein said first communication sub module, said second communication sub module and said third communication sub module are respectively arranged in said central server, said logical sub module and said storage server.

14. The device for four-dimensional visualization of medical images according to claim 12, wherein a medical image file uploaded by said client is received and analyzed and said medical image file and an analyzing result are saved to said storage server by said central server of said control module.

15. The device for four-dimensional visualization of medical images according to claim 14, wherein types of said medical image file comprises: file formats of Digital Imaging and Communications in Medicine, RawData, Statistical Parametric Mapping, Neuroimaging Informatics Technology Initiative, Interfile, Analyze, Portable Network Graphics, MINC, BMP, and JPEG.

16. The device for four-dimensional visualization of medical images according to claim 14, wherein segmentation, registration, modeling and reconstruction processing are performed on said medical image file according to said user requests by said logical sub module.

17. The device for four-dimensional visualization of medical images according to claim 16, wherein cross-sectional tomographic images are extracted according to said medical image file and said cross-sectional tomographic images are stored to said storage server by said storage sub module.

18. The device for four-dimensional visualization of medical images according to claim 17, wherein said logical sub module comprises rendering core sub module, network communication sub module and logical management sub module which communicate with each other, wherein reconstruction calculation is performed on each frame of medical image file by said rendering core sub module, information is fed back to said client and interactive request messages are sent to said logical management sub module by said network communication sub module, and said interactive request messages are classified and sorted and useless or repeated interactive request messages are eliminated by said logical management sub module.

19. The device for four-dimensional visualization of medical images according to claim 10, wherein said control module, said communication module and said processing module are all constructed on a same server side, said client and said server side are independent of each other, and said client communicates with said server side via said control module and said communication module.

20. The device for four-dimensional visualization of medical images according to claim 19, wherein medical image data is transmitted between said client and said server side in forms of compressed data and messages.

21. The device for four-dimensional visualization of medical images according to claim 10, wherein user requests are sent to said control module by said client via said interconnection unit, said user requests are received and data is fed back to said client by said control module, and different user requests are distributed to said storage server, said cache server and said logical sub module by said control module simultaneously.

22. The device for four-dimensional visualization of medical images according to claim 10, wherein said client comprises a mobile phone, a tablet and a desktop computer.

23. The device for four-dimensional visualization of medical images according to claim 10, wherein said storage sub module and logical sub module in said processing module are formed by using a number of servers communicatively connected with each other, and said storage sub module and said logical sub module are constructed on different servers and operate independently of each other.

24. The device for four-dimensional visualization of medical images according to claim 10, wherein said processing module is provided with a resource monitoring sub module which communicates with said interconnection unit in said perception module.

25. The device for four-dimensional visualization of medical images according to claim 10, wherein said cache server is a key-value storage database.
